# EUROPÄISCHE PATENTSCHRIFT

(11) **EP 0 761 244 B1**
(45) Veröffentlichungstag und Bekanntmachung des Hinweises auf die Patenterteilung: **11.07.2001**
(21) Anmeldenummer: 96114225.4
(22) Anmeldetag: 05.09.1996
(51) Int. Cl.: A61L 33/00

(54) **Verfahren zum Anlagern und Immobilisieren von Heparin auf anorganischen Substratoberflächen von kardiovaskulären Implantaten**
Method for binding and immobilizing of heparin to inorganic substrate surfaces of cardiovascular implants
Procédé pour lier et fixer de l'héparine à la surface des substrats inorganiques d'implants cardiovasculaires

(30) Priorität: 12.09.1995 DE 19533682
(43) Veröffentlichungstag der Anmeldung: 12.03.1997
(73) Patentinhaber: BIOTRONIK Mess- und Therapiegeräte GmbH & Co Ingenieurbüro Berlin, 12359 Berlin (DE)
(72) Erfinder: Amon, Michael, 90556 Cadolzburg (DE); Bolz, Armin, Dr., 91054 Buckenhof (DE); Müssig, Dirk, 90439 Nürnberg (DE)
(74) Vertreter: Hübner, Gerd, Dipl.-Phys.

(56) Entgegenhaltungen:
- US-A- 3 677 795
- US-A- 4 265 927
- US-A- 5 002 582

## Beschreibung

Die Erfindung betrifft ein Verfahren zum Anlagern und Immobilisieren von Heparin auf anorganischen Substratoberflächen von kardiovaskulären Implantaten, wie Herzklappen-Implantaten oder alloplastischen Gefäßwandstützen, die im Fachjargon üblicherweise als "Stents" bezeichnet werden.

Zum Hintergrund der Erfindung ist auszuführen, daß bei der Einpflanzung von kardiovaskulären Implantaten generell das Problem einer akuten Thrombenbildung an der Oberfläche dieser Implantate besteht, da das damit in Kontakt kommende Blut zu Koagulation neigt. Um nun die Gefahr einer akuten Thrombenbildung zu reduzieren, ist es üblich, dem Patienten Antikoagulantien, z.B. Heparin-Präparate in hohen Dosierungen etwa durch Einspritzen in den Blutkreislauf zu verabreichen. Diese Art von Therapie ist jedoch mit unerwünschten Nebenwirkungen verbunden und daher grundsätzlich problematisch.

Ein Ansatz zur Lösung dieser Problematik besteht darin, gerinnungshemmende Medikamente auf der Oberfläche der Implantate chemisch zu binden. Damit wird das z.B. Heparin-Präparat, das auf katalytische Weise eine Koagulation des Blutes verhindert oder zumindest reduziert, nur dort eingesetzt, wo diese Antikoagulationseigenschaften benötigt werden, nämlich im Kontaktbereich des Implantates mit dem Blut.

Es sind nun technische Verfahren zur Belegung organischer Materialien mit Antikoagulatien bekannt (siehe beispielsweise B. Seifert et al "Heparinisierte Polylaktide als bioresorbierbare hämokompatible Biomaterialien" in Biomedizinische Technik 1994; 39: S. 103 bis 104). Speziell im Zusammenhang mit Brustkorb-Drainagekathetem aus Polyvinylchlorid ist es bekannt, an das PVC-Material einen photoaktiven Polyacrylamid-Heparin-(Photo-PA/HEP-)-Komplex zu binden. Diese Photo-PA/HEP-Beschichtung führt zu einem Katheter mit einer benetzbaren Oberfläche und verbesserten Blutkontakteigenschaften. Spuren von Heparin werden dabei freigesetzt und letzteres gleichzeitig kovalent an die Oberfläche des Katheters gebunden. Bei einem derartig ausgerüsteten Brustkorb-Drainage-Katheter ist die Blut-Klumpen- und Thrombosebildung in dem Bereich minimiert, in dem der Katheter mit dem sich im Brustraum ansammelnden Blut in Kontakt steht. Die vorstehenden Erkenntnisse ergeben sich aus dem Fachbeitrag zum "Surfaces in Biomaterials Symposium" von P. Gingras et al "Surface Modification of Poly(Vinyl Chloride) Chest Drainage Catheters with Anticoagulants for passive Drug Release".

Da nun die eingangs genannten kardiovaskulären Implantate aus anorganischen Materialien, wie z.B. Tantal, einer Titanlegierung, medizinischem Stahl oder pyrolytischem Kohlenstoff bestehen bzw. eine anorganische Beschichtung beispielsweise aus amorphem Siliziumkarbid aufweisen, besteht das Problem ein entsprechendes Verfahren zu finden, um Heparin-Präparate auf den genannten anorganischen Werkstoffen zu binden, damit kardiovaskuläre Implantate antikoagulative Eigenschaften aufweisen können.

Die Lösung dieses Problems ist im Anspruch 1 angegeben. Das demgemäß vorgeschlagene Verfahren weist folgendes Verfahrensschritte auf:
- Aktivierung der anorganischen Substratoberfläche durch Anätzen,
- Anlagern einer photoaktiven Benzophenonverbindung unter UV-Bestrahlung als Spacer mit einer Aminoschutzgruppe auf der aktivierten Substratoberfläche,
- Abspaltung der Aminoschutzgruppe mit einer wasserfreien Piperidinlösung, und
- kovalente Peptid-Bindung von Heparin an die freiliegenden und reaktionsfähigen Aminogruppen der Substratoberfläche durch deren Beaufschlagung mit einer wässrigen Heparin-Lösung.

Auf diese Weise kann ein kardiovaskuläres Implantat mit einer anorganischen Oberfläche hergestellt werden, an der biologisch aktives Heparin mit einer Oberflächenkonzentration von typischerweise 50 mIU/cm² angelagert ist. Aufgrund der kovalenten Peptid-Bindung des Heparins bleibt dessen Aktivität in vitro über Tage und Wochen unverändert. Die Heparin-Aktivität hemmt in positiver Weise die Blutgerinnung an den kardiovasulären Implantaten, was durch um den Faktor 3,5 bis 4 gegenüber unbehandelten Proben verlängerte Reaktions- und Gerinnselbildungszeiten bei der Thrombelastographie verifiziert werden kann.

Die Ansprüche 2 bis 8 kennzeichnen vorteilhafte Weiterbildungen des erfindungsgemäßen Verfahrens. So kann das Heparin einerseits auf einer aktivierten Substratoberfläche auf Metall- oder Kohlenstoff-Basis direkt, andererseits aber auch auf der aktivierten Substratoberfläche einer amorphen Siliziumkarbid-Beschichtung des Implantats deponiert werden. Derartige amorphe Siliziumkarbid-Beschichtungen haben in jüngster Zeit verstärkte Aufmerksamkeit als physiologisch günstige Beschichtungsart erfahren, deren positive Eigenschaften durch die hier beanspruchte Anlagerung und Immobilisierung von Heparin im Hinblick auf den erfindungsgemäß vorgesehenen Einsatzzweck noch stark verbessert werden können.

Die bevorzugtermaßen vorgenommene Aktivierung der Substratoberfläche durch Anätzen mit Fluorwasserstoff-Säure führt insbesondere im Zusammenhang mit einer amorphen Siliziumkarbid-Beschichtung zu einer wirksamen Protonierung der Oberfläche, was die Effektivität des weiteren Anlagerungsmechanismus unterstützt.

Die als photoaktive Benzophenon-Verbindung bevorzugt verwendete Fmoc-p-Bz-Phe-OH-Lösung in N,N'-Dimethylformamid (DMF) beruht auf einem handelsüblichen Produkt Fmoc-p-Bz-Phe-OH, was den Herstellungsvorgang naturgemäß vereinfacht und rationeller gestaltet.

Die Ansprüche 6 bis 8 beziehen sich auf bevorzugte Verfahrensparameter im Zusammenhang mit der verwendeten wässrigen Heparin-Lösung. Nähere Informationen darüber sind der Beschreibung des Ausführungsbeispiels entnehmbar.

Die Ansprüche 9 und 10 beziehen sich auf ein kardiovaskuläres Implantat, das mit Hilfe des erfindungsgemäßen Verfahrens hergestellt ist, so daß Heparin als gerinnungshemmender Stoff mittels einer kovalenten Peptid-bindung auf der Substratoberfläche angelagert und immobilisiert ist. Bevorzugtermaßen ist dabei die Substratoberfläche durch eine Beschichtung aus amorphem Siliziumkarbid gebildet.

Weitere Merkmale, Einzelheiten und Vorteile der Erfindung ergeben sich aus dem folgenden

### Ausführungbeispiel:

Es ist vorwegzuschicken, daß das erfindungsgemäße Verfahren anhand der Anlagerung und Immobilisierung von Heparin auf einer Siliziumkarbid(a-SiC:H)-Beschichtung (Probenfläche aus Tantal mit einer Beschichtung aus amorphem Siliziumkarbid, Probengröße 5 x 10 x 1 mm³) beschrieben wird. Die entsprechende Vorgehensweise und die mit der Probe erhaltenen Ergebnisse, wie sie im folgenden noch näher erläutert werden, können jedoch ohne weiteres auf die Anlagerung und Immobilisierung von Heparin auf Stents übertragen werden, die mit einer Siliziumkarbid-Beschichtung versehen sind.

Zur Verfahrensweise ist auszuführen, daß die Siliziumkarbid-Oberfläche zunächst mit 40%-iger Fluorwasserstoff-Säure angeätzt wird. Dieses Anätzen führt zur Anlagerung von Protonen an der Oberfläche, was sich durch übliche infrarotspektroskopische Untersuchungen der a-SiC:H-Oberfläche ermitteln läßt.

Danach wird die Probenoberfläche mit Wasser gespült und die Probe in 2 ml einer 20 x 10⁻⁶-molaren Fmoc-p-Bz-Phe-OH-Lösung in N,N'-Dimethylformamid (DMF) inkubiert. Die als photoaktive Spacer-Substanz wirksame Fmoc-p-Bz-Phe-OH-Lösung ist als handelsübliches Produkt "Fmoc-p-Bz-Phe-OH", Produktnummer B 2220 der Firma "Bachem Biochemica GmbH", Heidelberg zu beziehen. Die Reduktion des Benzophenons wird durch die Bestrahlung mit UV-Licht eingeleitet. Nach der UV-Bestrahlung wird die Reaktionslösung abgegossen und die Probe mehrmals mit destilliertem Wasser gespült.

Der nächste Schritt umfaßt die Abspaltung der Fmoc-Schutzgruppe mit 25%-iger Piperidinlösung in DMF. An der nun freiliegenden und reaktionsfähigen Aminogruppe erfolgt die Bindung des Heparins. Dazu wird die Probe in 1 ml einer wäßrigen Lösung, bestehend aus 10.000 IU Heparin und 50 mg N-(3-Dimethylaminopropyl)-N'-ethylcarbodiimid-hydrochlorid für einige Stunden bei Raumtemperatur inkubiert. Diese Reaktion wird durch das Abfritten der Reaktionslösung beendet.

Durch die vorstehend erläuterte Verfahrensweise wird auf der Siliziumkarbid-Oberfläche ein Siliziumkarbid-Spacer-Heparin-Komplex aufgebaut, wie er der folgenden Strukturformel entspricht.

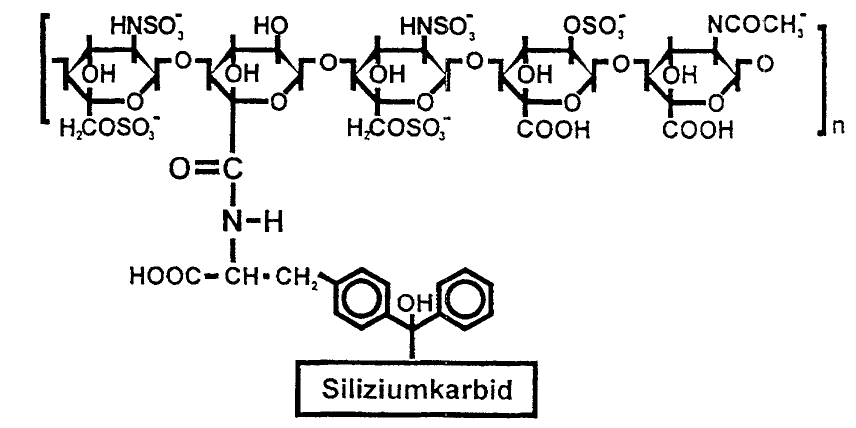

Die Oberflächenkonzentration des biologisch aktiven Heparins auf der Probe kann mittels einschlägiger biochemischer Verfahren zu 50 mIU/cm² bestimmt werden. Derartige Proben wurden weiterhin einem intensiven Spülvorgang in physiologischer Kochsalzlösung unterzogen, um das Verhalten der Heparin-Beschichtung im Blutstrom zu simulieren. Die entsprechenden Versuche ergaben, daß die biologische Aktivität auch nach einer Spülzeit von 14 Tagen nicht abnimmt. Dies bedeutet, daß praktisch eine 100%-ige Immobilisierung des Heparins auf der Siliziumkarbid-Oberfläche stattfindet.

Weiterhin wurde der Einfluß der Heparin-beladenen Beschichtung auf die Blutgerinnung durch den Vergleich der Thrombelastogramme von Proben mit Siliziumkarbid-Beschichtung ohne und mit Heparin-Anlagerung bestimmt. Die Thrombelastographie lieferte Reaktionszeiten und Gerinnselbildungszeiten, die bei Proben mit Heparin-Anlagerung um den Faktor 3,5 bzw. 4 höher lagen als bei Siliziumkarbid-beschichteten Proben ohne Heparin-Anlagerung. Diese Verzögerung der Blutgerinnung belegt, daß die Siliziumkarbid-Beschichtung mit Heparin-Anlagerung aufgrund ihrer biologischen Aktivität die Blutgerinnung an kardiovaskulären Implantaten hemmt.

## Patentansprüche

1. Verfahren zum Anlagern und Immobilisieren von Heparin auf anorganischen Substratoberflächen von kardiovaskulären Implantaten, wie Herzklappen-Implantaten oder alloplastischen Gefäßwandstützen, mit folgenden Verfahrensschritten:
- Aktivierung der anorganischen Substratoberfläche durch Anätzen,
- Anlagern einer photoaktiven Benzophenonverbindung mit einer Aminoschutzgruppe unter UV-Bestrahlung als Spacer auf der aktivierten Substratoberfläche,
- Abspaltung der Aminoschutzgruppe mit einer wasserfreien Piperidinlösung, und
- kovalente Peptid-Bindung von Heparin an die freiliegenden und reaktionsfähigen Aminogruppen der Substratoberfläche durch deren Beaufschlagung mit einer wässrigen Heparin-Lösung.

2. Verfahren nach Anspruch 1, dadurch gekennzeichnet, daß Heparin auf einer aktivierten Substratoberfläche aus Tantal, Titanlegierung, medizinischem Stahl oder pyrolytischem Kohlenstoff angelagert und immobilisiert wird.

3. Verfahren nach Anspruch 1, dadurch gekennzeichnet, daß Heparin auf der aktivierten Substratoberfläche einer amorphen Silizumkarbid-Beschichtung des Implantates angelagert und immobilisiert wird.

4. Verfahren nach einem der Ansprüche 1 bis 3, dadurch gekennzeichnet, daß die Aktivierung der Substratoberfläche durch Anätzen mit Fluorwasserstoff-Säure, vorzugsweise mit 40%iger Fluorwasserstoff-Säure erfolgt.

5. Verfahren nach einem der Ansprüche 1 bis 4, dadurch gekennzeichnet, daß als photoaktive Benzophenonverbindung eine Fmoc-p-Bz-Phe-OH-Lösung in N,N'-Dimethylformamid verwendet wird.

6. Verfahren nach einem der Ansprüche 1 bis 5, dadurch gekennzeichnet, daß die wässrige Heparin-Lösung aus Heparin mit N-(3-Dimethylaminopropyl)-N'-ethylcarbodiimid-hydrochlorid in Wasser besteht.

7. Verfahren nach Anspruch 6, dadurch gekennzeichnet, daß die wässrige Heparin-Lösung bezogen auf 1 ml Lösungsvolumen 10.000 IU Heparin und 50 mg N-(3-Dimethylaminopropyl)-N'-ethylcarbodiimid-hydrochlorid enthält.

8. Verfahren nach Anspruch 6 oder 7, dadurch gekennzeichnet, daß die Substratoberfläche mit der Heparin-Lösung für einen Zeitraum von 2 bis 6 Stunden bei Raumtemperatur beaufschlagt wird.

9. Kardiovaskuläres Implantat, wie künstliche Herzklappe oder alloplastische Gefäßwandstütze, hergestellt nach einem Verfahren gemäß einem der Ansprüche 1 bis 8 und mit einer anorganischen Substratoberfläche, dadurch gekennzeichnet, daß Heparin mittels einer kovalenten Peptid-bindung auf der Substratoberfläche angelagert und immobilisiert ist.

10. Kardiovaskuläres Implantat nach Anspruch 9, dadurch gekennzeichnet, daß die Substratoberfläche durch eine Beschichtung aus amorphem Siliziumkarbid gebildet ist.

## Claims

1. A method of attaching heparin to, and immobilizing it on, inorganic substrate surfaces of cardiovascular implants such as cardiac valves or alloplastic vessel wall supports, comprising the following steps:
- activation of the inorganic substrate surface by etching,
- attachment, by exposure to ultraviolet light, of a photoactive benzophenone compound with an amino protective group as a spacer to the activated substrate surface,
- splitting off of the amino protective group by the aid of a non-aqueous piperidine solution, and
- covalent peptide-bonding of heparin to the free and reactive amino groups of the substrate surface by an aqueous heparin solution acting on the amino groups.

2. A method according to claim 1, characterized in that heparin is attached to an activated substrate surface of tantalium, a titanium alloy, medical steel or pyrolytic carbon, where it is immobilized.

3. A method according to claim 1, characterized in that heparin is attached to the activated substrate surface of an amorphous silicon carbide coating of the implant, where it is immobilized.

4. A method according to one of the claims 1 to 3, characterized in that the activation of the substrate surface takes place by etching by means of aqueous hydrofluoric acid, preferably a 40 per cent aqueous hydrofluoric acid.

5. A method according to one of claims 1 to 4, characterized in that a Fmoc-p-Bz-Phe-OH solution in N,N'-dimethyl formamide is used as the photoactive benzophenone compound.

6. A method according to one of claims 1 to 5, characterized in that the acqueous heparin solution consists of heparin with N-(3-dimethylaminopropyl)-N'-ethylcarbodiimide-hydrochloride in water.

7. A method according to claim 6, characterized in that the aqueous heparin solution, related to 1 ml solution volume, contains 10,000 IU heparin and 50 mg N-(3-dimethylaminopropyl)-N'-ethylcarbodiimide-hydrochloride.

8. A method according to claim 6 or 7, characterized in that the heparin solution acts on the substrate surface for a period of 2 to 6 hours at ambient temperature.

9. A cardiovascular implant, such as an artificial cardiac valve or alloplastic vessel wall support, produced in accordance with a method according to one of claims 1 to 8 and having an inorganic substrate surface, characterized in that heparin is attached to, and immobilized on, the substrate surface by means of a covalent peptide bond.

10. A cardiovascular implant according to claim 9, characterized in that the substrate surface is formed by a coating of amorphous silicon carbide.

## Revendications

1. Procédé destiné à la fixation et à l'immobilisation d'héparine sur des surfaces de substrat inorganique d'implants cardiovasculaires tels que des implants de valvules cardiaques, ou des supports alloplastiques de parois vasculaires, comportant les phases de procédé suivantes :
- activation de la surface du substrat inorganique par attaque chimique,
- fixation par rayonnement UV sur la surface activée du substrat d'un composé de benzophénone photoactif en tant qu'espaceur avec un groupe amino-protecteur,
- élimination du groupe amino-protecteur avec une solution anhydre de pipéridine, et
- liaison peptidique covalente d'héparine aux groupes amino libérés et réactifs de la surface du substrat par application d'une solution aqueuse d'héparine.

2. Procédé selon la revendication 1, caractérisé en ce que de l'héparine est fixée et immobilisée sur une surface activée d'un substrat en tantale, en alliage de titane, en acier médical ou en carbone pyrolytique.

3. Procédé selon la revendication 1, caractérisé en ce que de l'héparine est fixée et immobilisée sur la surface activée d'un substrat d'un revêtement au carbure de silicium amorphe de l'implant.

4. Procédé selon l'une quelconque des revendications 1 à 3, caractérisé en ce que l'activation de la surface du substrat est effectuée par une attaque chimique avec de l'acide fluorhydrique, de préférence avec de l'acide fluorhydrique à 40 %.

5. Procédé selon l'une quelconque des revendications 1 à 4, caractérisé en ce que, en tant que composé de benzophénone photoactif, il est utilisé une solution de Fmoc-p-Bz-Phe-OH dans du N, N'-diméthylformamide.

6. Procédé selon l'une quelconque des revendications 1 à 5, caractérisé en ce que la solution aqueuse d'héparine est composée d'héparine avec du chlorhydrate de N-(3-diméthylaminopropyl)-N'-éthylcarbodiimide dans de l'eau.

7. Procédé selon la revendication 6, caractérisé en ce que, rapporté à 1 ml de volume de solution, la solution aqueuse d'héparine contient 10.000 UI d'héparine et 50 mg de chlorhydrate de N-(3-diméthylaminopropyl)-N'-éthylcarbodiimide.

8. Procédé selon la revendication 6 ou 7, caractérisé en ce que la solution d'héparine est appliquée à température ambiante sur la surface du substrat pendant une durée de 2 à 6 heures.

9. Implant cardiovasculaire, tel que valvule cardiaque artificielle ou support alloplastique de parois vasculaires, fabriqué d'après un procédé selon l'une quelconque des revendications 1 à 8, et comportant une surface de substrat inorganique, caractérisé en ce que de l'héparine est fixée et immobilisée sur la surface du substrat au moyen d'une liaison peptidique covalente.

10. Implant cardiovasculaire selon la revendication 9, caractérisé en ce que la surface du substrat est constituée d'un revêtement au carbure de silicium.
